# EUROPEAN PATENT APPLICATION

(11) **EP 3 427 753 A1**
(43) Date of publication of application: **16.01.2019**
(21) Application number: 18182982.1
(22) Date of filing: 11.07.2018
(51) Int. Cl.: A61K 45/06, A61K 31/135, A61K 31/55, A61P 13/12

(54) **COMBINATION OF ANTAGONISTS OF THE VASOPRESSIN RECEPTOR AND CALCIOMIMETICS, COMPOSITION AND USE THEREOF FOR THE TREATMENT OF POLYCYSTIC KIDNEY**

(30) Priority: 14.07.2017 IT 201700079551
(71) Applicant: Università degli Studi di Bari "Aldo Moro", 70121 Bari (BA) (IT)
(72) Inventor: VALENTI, Giovanna, 70125 Bari (BA) (IT); TAMMA, Grazia, 70125 Bari (BA) (IT); DI MISE, Annarita, 70125 Bari (BA) (IT); RANIERI, Marianna, 70125 Bari (BA) (IT)
(74) Representative: Trupiano, Federica

(57) **Abstract**

Combination of antagonists of the vasopressin receptor and calciomimetics and composition comprising it, and its use for the prevention and/or treatment of polycystic kidney.

## Description

### Abstract

Combination of antagonists of the vasopressin receptor and calciomimetics and composition comprising it, and its use for the prevention and/or treatment of polycystic kidney.

### Technical Field

The polycystic kidney disease, known as polycystic kidney, is a genetic disorder of cystic type of which an autosomal dominant form, more common and typical in the adult, and a recessive autosomal form, more rare and present in the childhood, exist. In particular, the autosomal dominant form (ADPKD) is the most common lethal genetic disease and is the first cause of kidney failure worldwide.

The polycystic kidney disease is caused by mutations in the PDK1 or PDK2 genes encoding for polycystin 1 (PC1) and polycystin 2 (PC2), respectively. PC1 interacts with PC2 to form a multifunctional complex, i.e. a channel, regulating the passage of intracellular calcium. The calcium ion is an important messenger that, when entering the cell or when it is released by the intracellular deposits, triggers a number of cellular changes. When PC1 or PC2 are mutated, such a channel is not adequately operating and the signal induced by the calcium is altered. All of this translates into the formation of cysts in both kidneys.

The kidney cysts are roughly spherical formations having a liquid content, that develop in the context of the renal parenchyma and precisely in the glomerulus and in the proximal and distal tubules. The formation of the cysts compromises the normal kidney function, giving glomerular filtration problems, urine concentration and excretion of the same. At present there is no pharmacological cure for the disease. In fact, the pharmacological treatments that have been studied (i.e., the treatments given herein below as known art) exclusively aim at slowing the formation of cysts and/or have different disadvantages that impede their use.

The treatment with the tyrosine kinase EGF inhibitors was effective in some murine animal models only.

The treatment with the cyclin kinase inhibitors, and in particular with roscovitine, causes heavy side effects.

Another type of inhibitor tested for this disease is the mTOR inhibitor, protein-kinase regulating the growth, proliferation, motility and survival of the cells, protein synthesis and transcription. The mTOR inhibitors, on the long term, cause nephrotoxicity.

Statins are drugs that inhibit the synthesis of endogenous cholesterol and have been tested in patients affected by the polycystic kidney only to alleviate the symptoms of the disease. Last, the calciomimetics are drugs instead that act by activating the calcium receptors and causing an increase of intracellular calcium. At present, the calciomimetics are approved for the treatment of secondary hyperparathyroidism. However, the calciomimetics have the side effect of hypocalcemia, that in turn can induce psychic disorders of various kinds, arrhythmias, muscle spasms, convulsions, etc. in the patient.

To date, the only drug recognized and approved for its potential in slowing the enlargement of the cysts, and consequently the progression of the disease, is tolvaptan, approved drug for the treatment of hyponatraemia.

Tolvaptan (belonging to the class of vaptans) at the moment is the only drug accepted for the treatment of polycystic kidney and acts as a competitive antagonist of the vasopressin V2 receptors located at the basolateral membrane of the main cells of the renal collecting duct.

Physiologically, vasopressin regulates the reabsorption of water through the AQP2 water channel. Therefore, it follows that the use of an antagonist drug of the vasopressin acts by inhibiting the signaling pathway of the V2 receptor and involving frequent diuresis as a side effect.

Given that the aforementioned drugs are ineffective in the complete resolution of the disease, and/or given the heavy side effects they entail, there is the need to identify new drugs or combinations of drugs able to effectively treat the polycystic kidney, and which at the same time do not induce unpleasant side effects.

### Objects of the Invention

It is a purpose of the invention to provide a combination that acts as pharmacological therapy for the treatment of the polycystic kidney, and that at the same time limits the side effects associated with the use of the only drug that is currently recommended for the treatment of this disease.

It is also a purpose of the invention to provide a pharmaceutical composition that comprises said combination.

Finally, it is a purpose of the invention to provide a use for said combination and composition.

### Description of the Invention

It is an object of the present invention a combination of one or more antagonists of the vasopressin receptor and one or more calciomimetics.

In a preferred embodiment, said one or more antagonists of the vasopressin receptor are selected from the class of vaptans, even more preferably said antagonist of the vasopressin receptor is tolvaptan.

In another preferred embodiment, said calciomimetic is tecalcet.

In a particularly preferred embodiment, said antagonist of the vasopressin receptor is tolvaptan and said calciomimetic is tecalcet.

According the present invention, said combination is a fixed combination in which the antagonist of the vasopressin receptor is present in amounts between about 30% and about 80%, preferably between about 50% and about 60% to the weight of the combination, whereas said calciomimetic is present in amounts between about 20% and about 70%, preferably between about 40% and about 50% to the weight of the combination. Surprisingly, the combination of the invention showed an *in vitro* unexpected efficacy in the prevention, attenuation of the progression and in the treatment in general terms of the polycystic kidney, in particular the autosomal dominant polycystic kidney (ADPKD). In fact, the components of the combination according to the invention show a synergistic effect, not foreseeable a priori.

Furthermore, said combination allows significantly reducing the side effects caused instead by the use, in monotherapy, of the individual drugs that make up the combination of the invention, i.e. the high diuresis when said antagonist of the vasopressin receptor is tolvaptan and hypocalcemia when said calciomimetic is tecalcet.

In the patients affected by polycystic kidney, especially autosomal dominant polycystic kidney (ADPKD), the combination of the invention would slow the onset and progression of the cysts, resulting in a marked improvement, and sometimes even the resolution of the associated symptomatology.

The combination of the invention can be equal to, for example, 1:1 in a weight/weight ratio between the one or more antagonists of the vasopressin receptor and the one or more calciomimetics.

According to another of its aspects, object of the invention is a pharmaceutical composition comprising the combination of the invention in any of the embodiments thereof, and at least one pharmaceutically acceptable excipient.

Therefore, the components of the combination of the invention can be formulated, when in fixed combination one to another, in pharmaceutical compositions for the administration to mammals comprised the humans, for the treatment of the polycystic kidney, preferably autosomal dominant polycystic kidney (ADPKD).

Said at least one pharmaceutically acceptable excipient, as well as the ratios of said excipients to the active ingredients and the methods for preparing the pharmaceutical composition of the invention can appropriately be selected by the skilled in the art. Organic or inorganic substances, or solid or liquid substances, can be used as pharmaceutical excipients.

Generally, the excipients can be incorporated in an amount ranging from 1% by weight to 99% by weight, based on the weight of the active ingredient, in this case of the fixed combination according to the invention.

According to a further embodiment, the pharmaceutical composition of the invention can be further added with a characteristic active ingredient for another medicament, still for the treatment of the polycystic kidney.

The type of pharmaceutical composition can be provided under any formulation form, preferably for the oral administration.

In a preferred embodiment, the pharmaceutical composition of the invention is for oral route, and can be, for example, in the form of oral tablet, capsule, powder, granulate, solution or suspension, and the like.

The composition of the invention can be prepared or produced by methods that are conventional and known to the skilled in the art.

The dose and administration frequency of the composition according to the invention can be appropriately selected depending on the patient conditions. The dosage can widely vary depending on the age, weight, health status of the patient, on the nature and severity of the condition, as well as the route of administration. According to the usual practice, the appropriate dosage for each patient is determined by the doctor.

According to the present invention, said antagonists of the vasopressin receptor in combination with said calciomimetic are used in the treatment of the polycystic kidney. Still according to the invention, said combination is a fixed combination.

Finally, it is an object of the invention the combination or composition of the invention for the use thereof as a medicament, preferably for the treatment of the polycystic kidney, even more preferably for the use in the treatment of the autosomal dominant form of the polycystic kidney (ADPKD).

According to another of its aspects, object of the invention is a method for treating the polycystic kidney providing the administration of said combination or composition according to the invention to a subject in the need thereof.

The surprising effect of the combination of the invention is due to the synergy of the individual components of the combination, i.e. the one or more antagonists of the vasopressin receptor and the one or more calciomimetics. In fact, as it will be shown in the Experimental Section, they act on two different alterations that are found in the renal cells of the patients affected by polycystic kidney, thus re-establishing the physiological condition.

Such alteration results as two events characterizing the pathogenesis and progression of the polycystic kidney, and are a) a reduction of the intracellular calcium levels and b) an intracellular increase of the second cAMP messenger.

As it could be seen in the Experimental Section, it has been demonstrated that the individual components of the combination of the invention act on the aforementioned alterations, and restore the physiological levels of intracellular calcium and cAMP.

### Description of the Figures

Figure 1 depicts the calcium ion levels in the two indicated cell types.
Figure 2 depicts the result of western blotting of the CaSR protein on the three indicated cell types.
Figure 3 depicts the results of immunofluorescence of the CaSR protein on the three indicated cell types.
Figure 4 depicts the intracellular calcium levels following the treatment with tecalcet in the three indicated cell types.
Figure 5 depicts the intracellular cAMP levels following the treatment with tecalcet in the indicated cell types.
Figure 6 depicts the intracellular cAMP levels following the treatment with tecalcet in the indicated cell type.
Figure 7 depicts the phosphorylation levels of the S6 protein following the treatment with tecalcet in the indicated cell types.
Figure 8 depicts the phosphorylation levels of the S6 protein following the treatment with tecalcet in the indicated cell type.
Figure 9 depicts the intracellular cAMP levels following the treatment with tolvaptan.
Figure 10 depicts the intracellular calcium levels following the treatment with tolvaptan.

### Experimental section

The experiments described from Example 1 to Example 5 have been carried out on i) ciPTEC human kidney cells of the proximal tubule isolated from healthy subjects (ciPTECwt), ii) on the same cells silenced for the gene encoding the polycystin 1 (ciPTE-PC1KO) and iii) on ciPTEC cells isolated from patients affected by ADPKD (ciPTEC-PC1Pt).

### Example 1

### ciPTE-PC1KO cellular model

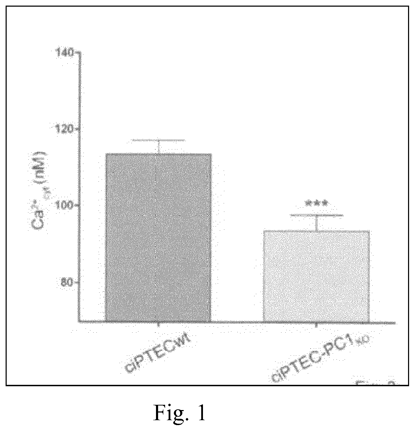

Calibration experiments of the intracellular calcium levels (Figure 1), that have demonstrated that under basal conditions the ciPTEC-PC1KO cells, in which the polycystin 1 has been stably silenced, have calcium levels that are significantly lower than the ciPTECwt cells isolated from healthy subjects (93.4 nM vs. 113.4 nM, P = 0.0004), have been carried out. Therefore, the ciPTEC-PC1KO cells are a valid model of human kidney cells of the proximal tubule of patients suffering from ADPKD.

### Example 2

### Presence of CaSR on ciPTECwt, ciPTEC-PC1KO and ciPTEC-PC1Pt cells

Calciomimetics conventionally act on the CaSR membrane receptor present on some cellular types, for example on the parathyroid cells, by sensitizing such a receptor in the presence of extracellular calcium ions.

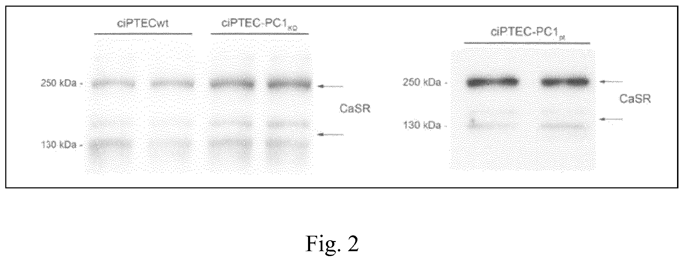

The expression by the ciPTECwt, ciPTEC-PC1KO and ciPTEC-PC1Pt cellular lines of CaSR has been verified through Western Blotting. From Figure 2, it can be noted that all of the cellular lines isolated from healthy subjects (ciPTECwt), the cells isolated from patients affected by ADPKD (ciPTEC-PC1Pt) and the ciPTEC-PC1KO cells, endogenously express the CaSR receptor.

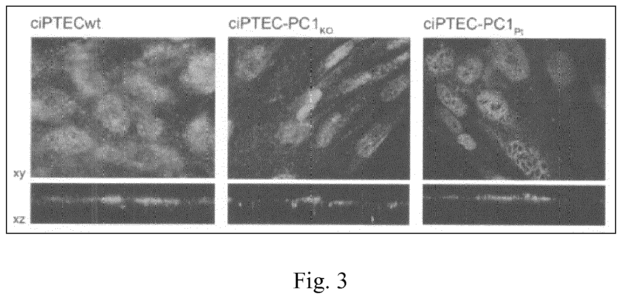

Therefore, the presence of such a receptor on the kidney cells isolated from healthy subjects has been demonstrated on the kidney cells isolated from patients suffering from ADPKD and on the ciPTEC-PC1KO cells. Furthermore, the location of the CaSR receptor has been detected at the level of the plasmatic membrane (Figure 3) through immunofluorescence experiments.

### Example 3

### Increase of the intracellular calcium levels following the treatment with calciomimetics

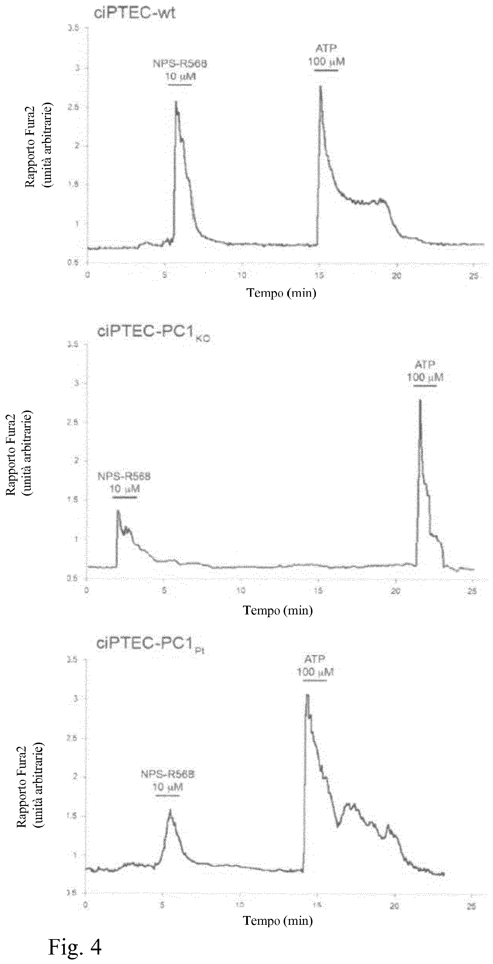

Three types of the aforementioned cellular lines have been treated with 10 µM calciomimetic tecalcet, and the result is that the ciPTEC-PC1Pt cells, isolated from patients suffering from the polycystic kidney disease, respond similarly to the ciPTECwt cells to the stimulation with the calciomimetic, thus leading to an increase of intracellular calcium levels (Figure 4). Such intracellular calcium levels, in the present example, have been detected thanks to the colorant Fura 2 that is sensitive to calcium.

Therefore, it has been demonstrated that the calciomimetic tecalcet exerts a positive action on one of the alterations present in the polycystic kidney disease, i.e. the decrease of intracellular calcium.

### Example 4

### Reduction of the intracellular cAMP levels following the treatment with calciomimetics

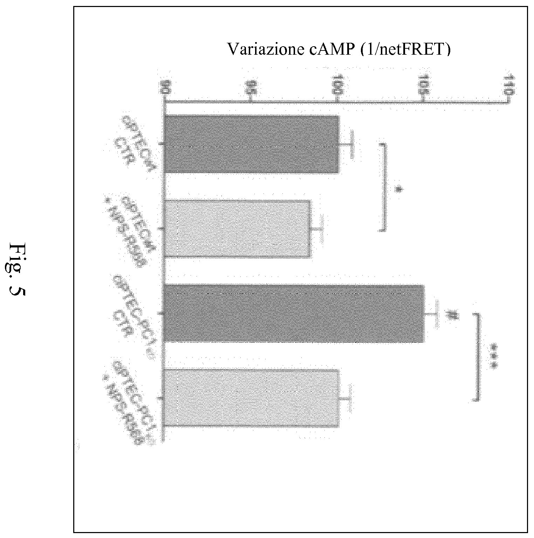

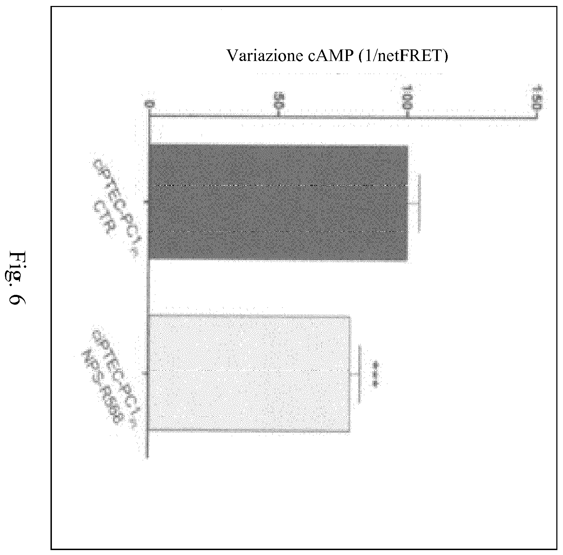

By the treatment of the aforementioned lines with the calciomimetic tecalcet, it has been demonstrated that the calciomimetics are also useful in controlling the cAMP cellular levels. In fact, at the concentration of 10 µM, tecalcet reduces the basal cAMP levels both in ciPTEC-PC1KO (Figure 5) and in ciPTEC-PC1Pt (Figure 6). Such cAMP levels have been measured through FRET.

Therefore, it has been demonstrated that the calciomimetic tecalcet also exerts a positive action on another of the alterations present in the polycystic kidney disease, i.e. the increase of intracellular cAMP.

### Example 5

### Regulation of the mTOR signaling pathway following the treatment with calciomimetics

It has also been demonstrated that the calciomimetic tecalcet allows the mTOR signaling pathway to be down-regulated.
mTOR is a serine/threonine kinase that, by the phosphorylation of proteins, regulates the cellular growth, proliferation and differentiation. In the context of the polycystic kidney disease, an up-regulation of mTOR is associated with the formation and development of the renal cysts.

In the present example, the activity of mTOR has been studied by detecting the phosphorylation levels of the S6 ribosomal protein.

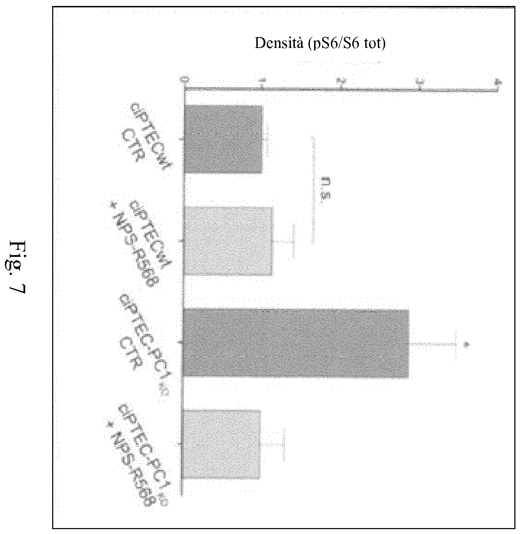

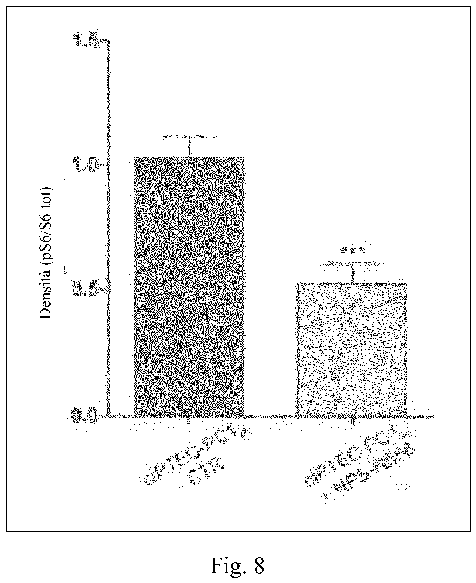

From Figures 7 and 8, it is clear that the activity of mTOR is up-regulated both in the ciPTEC-PC1KO cells and in the ciPTEC-PC1Pt cells. In fact, the basal levels of the S6 phosphorylation have been found significantly higher than those observed in the ciPTECwt cells isolated from the urinary sediment of healthy subjects.

By treating the aforementioned cells with the calciomimetic tecalcet at the concentration of 10 µM, the activity of mTOR is reduced, thus decreasing the S6 phosphorylation. Again, still from Figure 7, it is possible to note that the S6 phosphorylation levels are even brought back to comparable levels to those observed in the ciPTECwt cells.

### Example 6

### Reduction of the intracellular cAMP levels following the treatment with antagonists of the vasopressin receptor

The effects of the antagonists of the vasopressin receptor on the concentration of intracellular cAMP have been studied in the canine kidney cells MDCK endogenously expressing the V2 receptor.

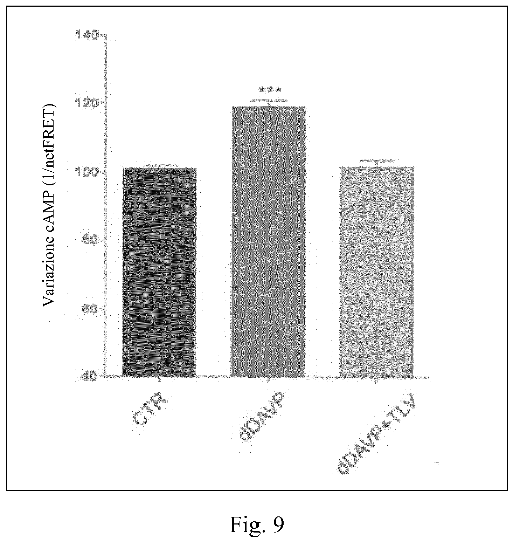

As it can be noted from Figure 9, it has been found that the treatment with tolvaptan reduces the cAMP levels induced by desmopressin (dDAVP, i.e. a synthetic version of vasopressin). Such a reduction of the cAMP levels has been detected through 1/netFRET.

### Example 7

### Increase of the intracellular calcium levels following the treatment with antagonists of the vasopressin receptor

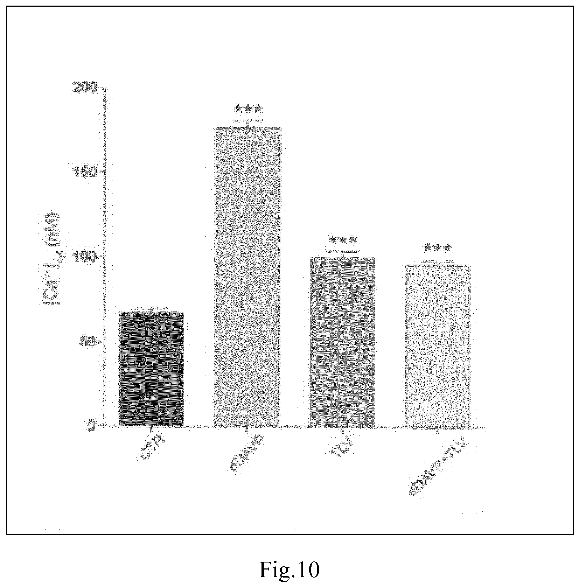

As it can be seen from Figure 10, tolvaptan, and in general the antagonists of the vasopressin receptor, not only lead to a reduction of the cAMP levels as demonstrated in the previous example, but further determine an increase of the intracellular calcium levels.

### Detailed Description of the Figures

### Figure 1: Calibration experiments of intracellular calcium levels.

Under basal conditions the ciPTEC-PC1KO cells, wherein PC1 has been silenced, show basal calcium levels that are lower than the ciPTECwt cells isolated from healthy subjects (93.4 nM vs. 113.4 nM, P = 0.0004).

### Figure 2: Expression of CaSR receptor in human kidney cells.

Western Blotting experiments detect the endogenous expression of the CaSR receptor.
- ciPTECwt isolated from healthy subjects and ciPTEC-PC1KO isolated from healthy subjects and silenced for PC1.
- ciPTEC-PC1Pt isolated from affected patients.

It is possible to highlight the monomeric form of the protein at 130 kDa, the glycosylated form at 160 kDa and the mature dimeric form at 250 kDa.

### Figure 3: Localization of the CaSR receptor at the plasmatic membrane level.

Immunofluorescence experiments in ciPTECwt, ciPTEC-PC1KO, ciPTEC-PC1Pt cells.

### Figure 4: Stimulation of the ciPTECwt, ciPTEC-PC1KO, ciPTEC-PC1Pt Cells withNPS-R568 calciomimetic.

The ciPTEC-PC1Pt cells respond similarly to the ciPTECwt cells to the stimulation with NPS-R568 calciomimetic, also called tecalcet, at a concentration of 10 µM. From the statistical analysis of the data it is clear that, with respect to the ciPTECwt cells, (90.68 ± 2.47% vs. ATP 100%, n = 40), also the ciPTEC-PC1KO cells (24.32 ± 2.59% vs. ATP 100%, n = 47) and those isolated from the ADPKD patients (29.51 ± 3.93% vs. ATP 100%, n = 30) significantly respond to NPS-R568, also called tecalcet, increasing the intracellular calcium levels. The intracellular calcium levels have been measured through the colorant Fura 2 that is sensitive to calcium.

### Figure 5: Effect of NPS-R568, also called tecalcet, on the Intracellular cAMP levels in ciPTEC-PC1KO cells.

At the concentration of 10 µM, the NPS-R568 calciomimetic, also called tecalcet, in addition to modulating the intracellular cAMP levels, reduces the basal cAMP levels in ciPTEC-PC1KO cells. The cAMP levels have been measured through 1/netFRET.

### Figure 6: Effect of NPS-R568, also called tecalcet, on the intracellular cAMP levels in ciPTEC-PC1Pt cells.

At the concentration of 10 µM, the NPS-R568 calciomimetic, also called tecalcet, in addition to modulating the intracellular cAMP levels, reduces the basal cAMP levels in ciPTEC-PC1Pt cells. The cAMP levels have been measured through 1/netFRET.

### Figure 7: Effect of NPS-R568, also called tecalcet, on the mTOR signaling in ciPTEC-PC1KO cells

The NPS-R568 calciomimetic, also called tecalcet, shows its efficacy in down-regulating the mTOR signaling pathway. The basal levels of the S6 phosphorylation are significantly higher in ciPTEC-PC1KO cells than those observed in the ciPTECwt cells. The treatment with NPS-R568, also called tecalcet, at the concentration of 10 µM, reduces the phosphorylation of S6.

### Figure 8: Effect of NPS-R568, also called tecalcet, on the mTOR signaling in ciPTEC-PC1Pt cells

The NPS-R568 calciomimetic, also called tecalcet, shows its efficacy in down-regulating the mTOR signaling pathway. The basal levels of the S6 phosphorylation are significantly higher in ciPTEC-PC1Pt cells than those observed in the ciPTECwt cells. The treatment with NPS-R568, also called tecalcet, at the concentration of 10 µM, reduces the phosphorylation of S6.

### Figure 9: Tolvaptan reduces the cAMP levels induced by vasopressin (dDAVP) in MDCK cells.

In cells treated with Tolvaptan (dDAVP + TLV) the cAMP levels induced by vasopressin are comparable to those of the control (CTR) and lower than those where the treatment with Tolvaptan (dDAVP) is not made. Thus, the treatment with Tolvaptan leads to a reduction of the intracellular cAMP levels. The cAMP levels have been measured through 1/netFRET.

### Figure 10: The treatment with Tolvaptan determines an increase of the intracellular calcium levels.

The treatment with Tolvaptan determines an increase of the intracellular calcium levels (TLV = 99.6 ± 4.1 nM; CTR = 67.06 ± 2.6 nM, P <0.0001).

## Claims

1. Combination of one or more antagonists of the vasopressin receptor and one or more calciomimetics.

2. Combination according to claim 1, **characterized in that** said one or more antagonists of the vasopressin receptor are selected from the class of vaptans.

3. Combination according to claim 1, **characterized in that** said antagonist of the vasopressin receptor is tolvaptan.

4. Combination according to claim 1, **characterized in that** said calciomimetic is tecalcet.

5. Combination according to claim 1, **characterized in that** said combination has 1:1 weight/weight ratio between said one or more antagonists of the vasopressin receptor and said one or more calciomimetics.

6. Pharmaceutical composition **characterized by** comprising said combination according to any one of the preceding claims, and at least one pharmaceutically acceptable excipient.

7. Pharmaceutical composition according to claim 6, **characterized in that** it can be administered by oral route.

8. Combination according to claim 1 for use as a medicament.

9. Combination according to claim 1 for use in the treatment of polycystic kidney.

10. Pharmaceutical composition according to claim 6 for use in the treatment of polycystic kidney.
